Europäisches Patentamt

European Patent Office

Office européen des brevets

(19)

(11) Veröffentlichungsnummer: **0 387 647**
**A2**

(12) **EUROPÄISCHE PATENTANMELDUNG**

(21) Anmeldenummer: **90104167.3**

(22) Anmeldetag: **03.03.90**

(51) Int. Cl.5: **A61K 9/107, A61K 47/20,**
**A61K 47/18, A61K 47/48**

(30) Priorität: **13.03.89 DE 3908047**

(43) Veröffentlichungstag der Anmeldung:
**19.09.90 Patentblatt 90/38**

(84) Benannte Vertragsstaaten:
**CH ES FR GB IT LI NL SE**

(71) Anmelder: **Desitin Arzneimittel GmbH**
**Weg beim Jäger 214**
**D-2000 Hamburg 63(DE)**

(72) Erfinder: **Matouschek, Reinhard, Dr.**
**Am Hehsel 13**
**D-2000 Hamburg 63(DE)**

(74) Vertreter: **UEXKÜLL & STOLBERG**
**Patentanwälte**
**Beselerstrasse 4**
**D-2000 Hamburg 52(DE)**

(54) **Hochdisperse pharmazeutische Zusammensetzung.**

(57) Es wird eine hochdisperse pharmazeutische Zusammensetzung beschrieben, die als Mikroemulsion vorliegt und durch Mischen von mit Ionenpaarbildnern modifiziertem sauren oder basischen Wirkstoff, Isoproylmyristat als Fettkomponente, Polyoxyethylen-fettsäureester und/oder Polyoxyethylenalkoholether als Tensid, Polyoxyethylen-glycerinfettsäureester als Cotensid und Wasser erhältlich ist. Die Zusammensetzung weist hohe Wirkstoffkonzentrationen auf und ist insbesondere zur nasalen, rektalen und transdermalen Applikation geeignet.

EP 0 387 647 A2

## Hochdisperse pharmazeutische Zusammensetzung

Die Erfindung betrifft eine hochdisperse pharmazeutische Zusammensetzung, die wirkstoffreich ist, als Mikroemulsion vorliegt und insbesondere nasal, rektal und transdermal appliziert werden kann.

Für die pharmazeutische Wirksamkeit insbesondere von äußerlich verabreichten pharmazeutischen Zusammensetzungen sind vor allem deren Gehalt an Wirkstoff sowie die Resorbierbarkeit dieses Wirkstoffes von der Haut oder den Schleimhäuten von besonderer Bedeutung. Die Resorbierbarkeit eines Wirkstoffes kann unter Umständen durch Einarbeitung desselben in sogenannte Mikroemulsionen als pharmazeutischer Träger erhöht werden. Unter Mikroemulsionen werden thermodynamisch stabile, einphasige Mehrkomponentenemulsionen verstanden, die auch bei längerer isothermer Lagerung im Gegensatz zu Makroemulsionen keine Auftrennung in makroskopisch sichtbare Phasen zeigen. Der breiten Verwendbarkeit von Mikroemulsionen als pharmazeutische Träger steht jedoch der Umstand entgegen, daß die bekannten Mikroemulsionen in vielen Fällen Komponenten hoher Toxizität enthalten und daher nicht appliziert werden können.

Aus der EP-A-278 660 sind wirkstofffreie Mikroemulsionen bekannt, die mindestens 20 Gew.% hydrophobe Ölphase, 0,01 bis 20 Gew.% hydrophile Phase, 0,01 bis 20 Gew.% quartäres Ammoniumsalz als kationaktives Tensid und eine ausreichende Menge Cotensid enthalten, um die Herstellung einer homogenen und klaren Mikroemulsion zu gewährleisten.

In der EP-A-255 485 sind transdermale Zusammensetzungen beschrieben, die mindestens einen Wirkstoff und eine Trägersubstanz enthalten, die eine Fettkomponente und zwei Lösungsmittel für den Wirkstoff umfaßt. Als Lösungsmittel werden mindestens ein Ester und mindestens ein Alkohol oder Glykol eingesetzt.

Aus der EP-A-214 501 sind Makroemulsionen zur Verabreichung von wenig wasserlöslichen hydrophoben Wirkstoffen bekannt. Sie sind aus einem hydrophoben Wirkstoff, einem aus natürlichen Pflanzenölen und halbsynthetischen Mono-, Di- oder Triglyceriden ausgewählten ölartigen Träger, einem Tensid, einem Cotensid, einem Ionenpaarbildner und Wasser zusammengesetzt. Als Ionenpaarbildner werden bei basischen Wirkstoffen gesättigte oder ungesättigte aliphatische oder aromatische $C_6$- bis $C_{20}$- Säuren und bei Verwendung von sauren Wirkstoffen pharmazeutisch akzeptable aliphatische oder aromatische Amine eingesetzt. Die ausschließlich zur intravenösen, intramuskulären, intraartikulären oder oralen Verabreichung vorgesehenen Zusammensetzungen müssen zur Bildung von homogenen Emulsionen bei Drucken von 552 bis 1034 bar homogenisiert werden.

In der DE-C-24 48 119 werden pharmazeutische Zubereitungen beschrieben, die parenteral oder oral in Tabletten- oder Kapselform verabreicht werden können. Die Zubereitungen enthalten einen wasserunlöslichen Wirkstoff mit einem tertiären protonierbaren N-Atom, $C_{14}$- bis $C_{24}$-Fettsäuren ein nichtionisches oberflächenaktives Mittel und einen pharmazeutischen Träger. Sie ergeben bei Zugabe zu Wasser klare Lösungen. Als nichtionische oberflächenaktive Mittel finden bevorzugt polyoxyethyliertes Pflanzenöl und Polyoxyethylensorbitanfettsäureester Anwendung. Als pharmazeutischer Träger werden z.B. Stärke, Methylcellulose, Magnesiumstearat und Laktose eingesetzt.

Der Erfindung liegt die Aufgabe zugrunde, als Mikroemulsionen vorliegende hochdisperse pharmazeutische Zusammensetzungen zur Verfügung zu stellen, die ohne Durchführung von Homogenisierungsoperationen bei hohen Drucken herstellbar sind, keine toxischen Komponenten enthalten, eine erhöhte Wirkstoffkonzentration aufweisen und insbesondere nasal, rektal und transdermal appliziert werden können.

Diese Aufgabe wird überraschenderweise durch die hochdisperse pharmazeutische Zusammensetzung gemäß den Ansprüchen 1 bis 11 und das Herstellungsverfahren gemäß den Ansprüchen 12 bis 13 gelöst.

Die erfindungsgemäße hochdisperse pharmazeutische Zusammensetzung ist dadurch gekennzeichnet, daß sie als Mikroemulsion vorliegt und durch Mischen von

a) saurem oder basischem Wirkstoff oder deren Salzen,

b1) in Gegenwart von saurem Wirkstoff oder dessen Salzen kationaktivem Ionenpaarbildner, dessen korrespondierende ungeladene Base einen pKs-Wert von mehr als 9 aufweist, in Form seiner Salze oder der korrespondierenden ungeladenen Base,

b2) in Gegenwart von basischem Wirkstoff oder dessen Salzen, anionaktivem Ionenpaarbildner, dessen korrespondierende ungeladene Säure einen pKs-Wert von weniger als 5 aufweist, in Form seiner Salze oder der korrespondierenden ungeladenen Säure,

c) Isopropylmyristat ,2- Octyldodecanol, Paraffinöl, Tocopherolnicotinat und/oder Triacetin als Fettkomponente,

d) Polyoxyethylenfettsäureester und/oder Polyoxyethylenfettalkoholether als Tensid,

e) Polyoxyethylenglycerinfettsäureester als Cotensid und

2

f) Wasser erhältlich ist.

Als saure Wirkstoffe können z.B. Diclofenac und/oder Ibuprofen und als basische Wirkstoffe z.B. Mutterkornalkaloide, vorzugsweise Dihydroergotamin, Dihydroergocristin und/oder Dihydroergotoxin, $\beta$-Sympathomimetica, vorzugsweise Salbutamol, Fenoterol, Ipratropium und/oder Terbutalin, und/oder $\beta$-Blocker, vorzugsweise Propranolol, Atenolol, Acebutolol und/oder Metoprolol, eingesetzt werden. Pharmazeutisch annehmbare Salze des sauren oder des basischen Wirkstoffs sind ebenfalls geeignet. Besonders bevorzugte Salze sind Dihydroergotaminmesilat, Salbutamolsulfat, Propranololhydrochlorid und Natriumdiclofenac.

Als weitere Komponente enthält die erfindungsgemäße Zusammensetzung einen Ionenpaarbildner. Unter der Bezeichnung "Ionenpaarbildner" wird in diesem Zusammenhang eine chemische Verbindung verstanden, die in der Lage ist, mit dem jeweiligen Wirkstoff oder dessen Salzen ein neutrales Ionenpaar zu bilden, das aus einem Ion des Wirkstoffs und einem gegensätzlich geladenen lipophilen Ion des Ionenpaarbildners aufgebaut ist. Es wird angenommen, daß der Wirkstoff durch die Bildung eines derartigen Ionenpaares lipophilisiert wird und damit schneller durch die Haut penetrieren kann. Mit den nachfolgend verwendeten Begriffen "kationenaktiver Ionenpaarbildner" und "anionaktiver Ionenpaarbildner" sind Ionenpaarbildner gemeint, die im ersten Fall ein lipophiles Kation und im zweiten Fall ein lipophiles Anion zur Bildung des Ionenpaares beisteuern. Die Art des eingesetzten Ionenpaarbildners hängt davon ab, ob es sich um einen sauren oder basischen Wirkstoff bzw. deren Salze handelt. Bei der Verwendung eines sauren Wirkstoffs oder von dessen Salzen wird ein kationaktiver Ionenpaarbildner in Form eines Salzes oder der korrespondierenden ungeladenen Base eingesetzt, wobei die korrespondierende ungeladene Base des kationaktiven Ionenpaarbildners einen pKs-Wert von mehr als 9 aufweist. Für den Fall, daß basische Wirkstoffe oder deren Salze eingesetzt werden, enthält die erfindungsgemäße Zusammensetzung einen anionaktiven Ionenpaarbildner in Form eines Salzes oder der korrespondierenden ungeladenen Säure, wobei die korrespondierende ungeladene Säure des anionaktiven Ionenpaarbildners einen pKs-Wert von weniger als 5 aufweist.

Als kationaktive Ionenpaarbildner können bevorzugt mit hydrophilen basischen Gruppen substituierte lipophile Verbindungen eingesetzt werden, die ohne basische Gruppe ein Molekulargewicht von mindestens 45, bevorzugt von mindestens 50, haben. Geeignete hydrohile basische Gruppen sind z.B. Trialkylammonium- und Tris-(oligooxyethyl)ammoniumgruppen. Ein konkretes Beispiel für eine mit hydrophiler basischer Gruppe substituierte lipophile Verbindung, die ohne basische Gruppe ein Molekulargewicht von mindestens 45 bzw. mindestens 50 hat, ist Cholinchlorid bzw. Benzyltrimethylammoniumchlorid. Besonders bevorzugte Beispiele für kationaktive Ionenpaarbildner sind kationaktive Tenside wie z.B. Tetraalkylammoniumsalze, Alkyl-bis-(oligooxyethyl)methylammoniumsalze, N-Fettsäurealkylamido-N-trimethylammoniumsalze, Tris-(oligooxyethyl)alkylammoniumsalze, Ester des Betains und/oder Ester des Cholins mit Fettsäuren. Ganz besonders bevorzugte kationaktive Ionenpaarbildner sind N-(2-hydroxyhexadecyl-1)-N,N-dimethyl-N-2-hydroxyethylammoniumchlorid (Handelsbezeichnung der wässrigen Lösung Dehyquart E) und Cetyltrimethylammoniumchlorid (Handelsbezeichnung der wässrigen Lösung Dehyquart® A). Die genannten kationaktiven Ionenpaarbildner können in Form ihrer Salze, z.B. Halogenide, Sulfate und Methansulfonate, oder als korrespondierende ungeladene Base eingesetzt werden. Voraussetzung ist jedoch stets, daß die korrespondierende ungeladene Base des eingesetzten kationenaktiven Ionenpaarbildners einen pKs-Wert von mehr als 9 aufweist.

Als anionaktive Ionenpaarbildner können bevorzugt mit hydrophi-len sauren Gruppen substituierte lipophile Verbindungen eingesetzt werden, die ohne saure Gruppe ein Molekulargewicht von mindestens 50 haben. Beispiele für hydrophile saure Gruppen sind Sulfat-, Sulfonsäure-, Taurin- und Glycingruppen. Beispiele für mit hydrophilen sauren Gruppen substituierte lipophile Verbindungen sind 2-Sulfobenzoate, Salicylsulfate und Taurocholate, insbesondere Natriumtaurocholat. Besonders bevorzugt können anionaktive Tenside, wie z.B. Alkylsulfate, insbesondere Natriumdodecylsulfat, Alkylpolyglykolethersulfate, Dialkylsulfosuccinate, insbesondere Natriumdioctylsulfo succinat und Alkylsulfonate als anionaktive Ionenpaarbildner in der erfindungsgemäßen Zusammensetzung verwendet werden. Die genannten anionaktiven Ionenpaarbildner können als Salze, z.B. Alkalisalze, oder in Form der korrespondierenden ungeladenen Säure eingesetzt werden. Es ist allerdings auf jeden Fall zu gewährleisten, daß anionaktive Ionenpaarbildner Verwendung finden, deren korrespondierende ungeladene Säure einen pKs-Wert von weniger als 5 hat. Werden als anionaktive Ionenpaarbildner mit lyophilen sauren Gruppen substituierte lipophile Verbindungen eingesetzt, die ohne saure Gruppe ein Molekulargewicht von weniger als 50 aufweisen, so müssen diese in der Lage sein, auch mit dem schlecht wasserlöslichen Wirkstoff Dihydroergotaminmesilat und den weiteren Bestandteilen eine klare Mikroemulsion bilden zu können.

Anstelle der beiden Komponenten a) Wirkstoff oder dessen Salzen und b) Ionenpaarbildner kann auch ein Wirkstoffsalz eingesetzt werden, das als Gegenion zum Wirkstoffion ein von einem Ionenpaarbildner abgeleitetes lipophiles Ion enthält.

Wenn der anionaktive Ionenpaarbildner als Salz vorliegt, wird der basische Wirkstoff bevorzugt ebenfalls in Form seiner Salze eingesetzt. Ansonsten können Ionenpaarbildner und Wirkstoff sowohl als Salze als auch in der freien Form verwendet werden.

Die weiteren zum Erhalt der erfindungsgemäßen hochdispersen pharmazeutischen Zusammensetzung erforderlichen Komponenten sind 2-Octyldodecanol (Handelsbezeichnung Eutanol® G), Paraffinöl, Tocopherolnicotinat und/oder Triacetin, vorzugsweise Isopropylmyristat, als Fettkomponente, Polyoxyethylenfettsäureester und/oder Polyoxyethylenfettalkoholether, vorzugsweise Polyoxyethylensorbitanfettsäureester, und besonders bevorzugt Polyoxyethy len-20-sorbitan-monooleat (Handelsbezeichnung Tween® 80), Polyoxyethylen-20-sorbitan-monostearat (Handelsbezeichnung Tween®60) Polyoxyethylen- 20-sorbitanmonolaurat (Handelsbezeichnung Tween® 20), Polyoxyethylen-30-cetylstearylalkoholether (Handelsbezeichnung Atlas® G-4940) und/oder Polyoxyethylen-30-cetylstearylalkoholether (Handelsbezeichnung Eumulgin® B 3), als Tensid, Polyoxyethylenglycerinfettsäureester, vorzugsweise Polyoxyethylen-7-glycerylcocoat (Handelsbezeichnung Cetiol® HE), als Cotensid und Wasser.

Voraussetzung für den Erhalt der erfindungsgemäßen Zusammensetzung ist stets, daß Fettkomponente, Tensid, Cotensid und Wasser zusammen eine Mikroemulsion bilden können.

Die erfindungsgemäße Zusammensetzung ist bevorzugt durch Mischen von (a) 0,1 bis 11 Gew.% Wirkstoff oder dessen Salzen, (b) 0,05 bis 12 Gew.% Ionenpaarbildner, (c) 1 bis 25 Gew.% der Fettkomponente, (d) 5 bis 30 Gew.% des Tensids, (e) 5 bis 30 Gew.% des Cotensids und (f) 5 bis 70 Gew.% Wasser erhältlich.

Das Gewichtsverhältnis von Ionenpaarbildner zu Wirkstoff kann in einem breiten Bereich von 0,15 bis 4,0 variieren und beträgt bevorzugt ungefähr 1.

Neben den beschriebenen wesentlichen Komponenten der erfindungsgemäßen pharmazeutischen Zusammensetzung können auch übliche Additive, z.B. Antioxidantien, wie Tocopherol, viskositätssteuernde Mittel, wie Glycerin, und/oder Geruchsverbesserer wie Citral eingesetzt werden, sofern diese die gewünschten Eigenschaften der Zusammensetzung nicht negativ beeinflussen. Es hat sich überraschenderweise gezeigt, daß höhere Konzentrationen, insbesondere 2 bis 5 Gew.%, an Vitamin E-Derivaten, insbesondere $\alpha$-Tocopherol, zu einer sehr guten chemischen Stabilität der in den Zusammensetzungen enthaltenden Wirkstoffe, wie z.B. Dihydroergotaminmesilat, führen.

Bei der Herstellung der als Mikroemulsion vorliegenden erfindungsgemäßen Zusammensetzung können die Komponenten oder Teile der Komponenten in beliebiger Reihenfolge insbesondere bei erhöhter Temperatur, bevorzugt bei 55 bis 90°C, miteinander gemischt werden. Insbesondere ist es möglich, den Wirkstoff und den Ionenpaarbildner mit einem Teil oder der Gesamtmenge des Wassers zu vermischen und zu dieser Vormischung die übrigen Komponenten in beliebiger Reihenfolge zuzugeben. Die für den jeweiligen Fall am besten geeignete Vorgehensweise kann in einfacher Weise mittels Routineexperimenten ermittelt werden.

Es ist bevorzugt, daß der pH-Wert der während des Mischens entstehenden wäßrigen Phasen beim Vorliegen von saurem Wirkstoff oder dessen Salzen im Bereich von pKs-Wert des sauren Wirkstoffs plus 1, bevorzugt plus 2, bis pKs-Wert der korrespondierenden ungeladenen Base des kationaktiven Ionenpaarbildners minus 1, bevorzugt minus 2, und beim Vorliegen von basischem Wirkstoff oder dessen Salzen im Bereich von pKs-Wert der korrespondierenden ungeladenen Säure des anionaktiven Ionenpaarbildners plus 1, bevorzugt plus 2, bis pKs-Wert des basischen Wirkstoffs minus 1, bevorzugt minus 2, liegt. Durch Zugabe von gängigen verdünnten Säuren oder Basen kann der pH-Wert der wäßrigen Phase innerhalb der oben angegebenen Bereiche gehalten werden. Auf diese Weise werden stark saure oder stark basische Bedingungen und damit eine mögliche Zersetzung des eingesetzten Wirkstoffes vermieden.

Die erhaltenen erfindungsgemäßen pharmazeutischen Zusammensetzungen liegen auch ohne Anwendung von besonderen Homogenisierungsoperationen als homogene klare Mikroemulsionen vor. Überraschenderweise können die Zusammensetzungen sogar beim Einsatz von schlecht wasserlöslichen Wirkstoffen hohe Wirkstoffmengen aufnehmen, womit sie sich für rektale, nasale oder transdermale Applikationen besonders eignen. Die ausgezeichnete pharmazeutische Verwendbarkeit der Zusammensetzungen wird außerdem dadurch gefördert, daß sie in hervorragender Weise von der Haut oder den Schleimhäuten resorbiert werden und ausschließlich aus Komponenten aufgebaut sind, die vom toxikologischen Standpunkt her unbedenklich sind.

Die Erfindung wird in den folgenden Beispielen näher erläutert.

| Beispiel 1 | |
|---|---|
| Dihydroergotaminmesilat | 3,2 Gew.% |
| Natriumdioctylsulfosuccinat | 2,2 Gew.% |
| Polyoxyethylen-20-sorbitanmonooleat (Tween 80) | 17,1 Gew.% |
| Polyoxyethylen-7-glycerylcocoat (Cetiol HE) | 16,3 Gew.% |
| Isopropylmyristat | 13,2 Gew.% |
| Wasser | 48,0 Gew.% |
| | 100,0 Gew.% |

Das Dihydroergotaminmesilat und Natriumdioctylsulfosuccinat wurden 30 Minuten lang in der Gesamtmenge des Wassers bei Zimmertemperatur gerührt. Anschließend wurde die Temperatur unter Rühren auf 55 bis 60°C erhöht, wobei die Suspension ausflockte. Es wurde nun unter kräftigem Rühren die Gesamtmenge von Polyoxyethylen-20-sobitanmonooleat, Polyoxyethylen-7-glycerylcocoat und Isopropylmyristat hinzugefügt und die Temperatur auf 75 bis 80°C angehoben. Nachdem sich alle Bestandteile aufgelöst hatten, wurde unter Rühren auf Zimmertemperatur abgekühlt.

| Beispiel 2 | |
|---|---|
| Dihydroergotaminmesilat | 3,2 Gew.% |
| Natriumdodecylsulfat | 1,2 Gew.% |
| Polyoxyethylen-20-sorbitanmonoleat (Tween 80) | 17,6 Gew.% |
| Polyoxyethylen-7-glycerylcocoat (Cetiol HE) | 16,7 Gew.% |
| Isopropylmyristat | 13,5 Gew.% |
| Tocopherol | 0,5 Gew.% |
| Wasser | 47,3 Gew.% |
| | 100,0 Gew.% |

Alle Bestandteile wurden zusammen 15 Minuten lang kräftig gerührt und anschließend solange auf 75 bis 80°C erhitzt, bis sich alle Bestandteile vollkommen aufgelöst hatten. Die heiße Lösung wurde danach unter Rühren auf Zimmertemperatur abgekühlt.

| Beispiel 3 | |
|---|---|
| Dihydroergotaminmesilat | 3,2 Gew.% |
| Natriumtaurocholat | 2,5 Gew.% |
| Polyoxyethylen-20-sorbitanmonoleat (Tween 80) | 25,9 Gew.% |
| Polyoxyethylen-7-glycerylcocoat (Cetiol HE) | 24,8 Gew.% |
| Isopropylmyristat | 20,0 Gew.% |
| Wasser | 23,6 Gew.% |
| | 100,0 Gew.% |

Das Dihydroergotaminmesilat und Natriumtaurocholat wurden in der gesamten Menge von Polyoxyethylen-20-sorbitanmonooleat, Polyoxyethylen-7-glycerylcocoat und Isopropylmyristat 15 Minuten lang kräftig gerührt. Anschließend wurde die Temperatur der Mischung auf 60°C angehoben, unter kräftigem Rühren die gesamte Menge des Wassers hinzugefügt und danach die entstandene Mischung solange auf 80°C erwärmt, bis eine klare Lösung entstanden war. Die Lösung wurde anschließend unter Rühren auf Zimmertemperatur abgekühlt.

| Beispiel 4 | |
|---|---|
| Salbutamolsulfat | 10,81 Gew.% |
| Natriumdioctylsulfosuccinat | 8,65 Gew.% |
| Polyoxyethylen-30-cetylstearylalkoholether (Eumulgin B 3) | 12,97 Gew.% |
| Polyoxyethylen-7-glycerylcocoat (Cetiol HE) | 21,08 Gew.% |
| Isopropylmyristat | 5,95 Gew.% |
| Wasser | 40,54 Gew.% |
| | 100,00 Gew.% |

Die Mischung aus Natriumdioctylsulfosuccinat, Salbutamolsulfat und Wasser wurde etwa 15 Minuten lang bei Zimmertemperatur gerührt und anschließend solange auf 80°C erwärmt, bis sich alles aufgelöst hatte. Getrennt davon wurde die Mischung aus Polyoxyethylen-30-cetylstearylalkoholether, Polyoxyethylen-7-glycerylcocoat und Isopropylmyristat unter Rühren auf 80°C erwärmt. Danach wurde die 80°C heiße Mischung aus Natriumdioctylsulfosuccinat, Salbutamolsulfat und Wasser in der Mischung aus Polyoxyethylen30-cetylstearylalkoholether, Polyoxyethylen-7-glycerylcocoat und Isopropylmyristat unter kräftigem Rühren aufgelöst. Die entstandene Mischung wurde unter Rühren auf Raumtemperatur abgekühlt.

| Beispiel 5 | |
|---|---|
| Propranololhydrochlorid | 6,3 Gew.% |
| Natriumdioctylsulfosuccinat | 9,5 Gew.% |
| Polyoxyethylen-30-cetylstearylalkoholether (Eumulgin B 3) | 15,8 Gew.% |
| Polyoxyethylen-7-glycerylcocoat (Cetiol HE) | 21,0 Gew.% |
| Isopropylmyristat | 5,3 Gew.% |
| Wasser | 42,1 Gew.% |
| | 100,0 Gew.% |

Die Herstellung erfolgte analog Beispiel 4 mit der Ausnahme, daß anstelle von Salbutamolsulfat Propranololhydrochlorid eingesetzt wurde.

| Beispiel 6 | |
|---|---|
| Natriumdiclofenac | 1,92 Gew.% |
| N-(2-Hydroxyhexadecyl-1)-N,N-dimethyl-N-2-hydroxyethylammoniumchlorid (Tensid in Dehyquart A) | 1,92 Gew.% |
| Polyoxyethylen-20-sorbitanmonooleat (Tween 80) | 19,23 Gew.% |
| Polyoxyethylen-7-glycerylcocoat (Cetiol HE) | 19,23 Gew.% |
| Isopropylmyristat | 19,23 Gew.% |
| Wasser | 38,47 Gew.% |
| | 100,0 Gew.% |

Eine wäßrige Lösung von N-(2-Hydroxyhexadecyl-1)-N,N-dimethyl-N-2-hydroxyethylammoniumchlorid (Gehalt 20 bis 30 Gew.%) wurde mit Wasser verdünnt, darin Natriumdiclofenac suspendiert und die Mischung unter Rühren auf 90°C erwärmt. Getrennt davon wurde eine Mischung aus Polyoxyethylen-20-sorbitanmonooleat, Polyoxyethylen-7-glycerylcocoat und Isopropylmyristat ebenfalls auf 90°C erwärmt. Danach wurde die Mischung aus Natriumdiclofenac, N-(2-Hydroxyhexadecyl-1)-N,N-dimethyl-N-2-hydroxyethylammoniumchlorid und Wasser langsam in die Mischung aus Polyoxyethylen-20-sorbitanmonooleat, Polyoxyethylen-7-glycerylcocoat und Isopropylmyristat unter kräftigem Rühren eingetragen und die gesamte Mischung auf Zimmertemperatur abgekühlt.

| Beispiel 7 | |
|---|---|
| Natriumdiclofenac | 1,74 Gew.% |
| Cetyltrimethylammoniumchlorid (Tensid in Dehyquart A) | 0,41 Gew.% |
| Polyoxyethylen-30-cetylstearylalkoholether (Eumulgin B 3) | 13,27 Gew.% |
| Polyoxyethylen-7-glycerylcocoat (Cetiol HE) | 20,41 Gew.% |
| Isopropylmyristat | 5,10 Gew.% |
| Wasser | 59,07 Gew.% |
| | 100,00 Gew.% |

Die Herstellung dieser pharmazeutischen Zusammensetzung erfolgte analog der in Beispiel 6 geschilderter Vorgehensweise, wobei Dehyquart E durch Dehyquart A und Tween 80 durch Eumulgin B 3 ersetzt wurden.

| Beispiel 8 | |
|---|---|
| Dihydroergotaminmesilat | 3,20 Gew.% |
| Natriumdodecylsulfat | 1,20 Gew.% |
| Tween® 80 | 15,93 Gew.% |
| Cetiol® HE | 15,93 Gew.% |
| Isopropylmyristat | 13,55 Gew.% |
| $\alpha$-Tocopherol | 02,39 Gew.% |
| Wasser, dest. | 47,80 Gew.% |
| Gesamt | 100,00 Gew.% |

Das Dihydroergotaminmesilat, das Natriumdodecylsulfat und das Wasser wurden 15 Minuten lang bei Zimmertemperatur gerührt und anschließend wurde die Mischung aus Tween® 80, Cetiol® HE, Isopropylmyristat und $\alpha$-Tocophrol hinzugefügt. Die Mischung aus sämtlichen Bestandteilen der Formulierung wurde unter Rühren auf 80°C erwärmt. Anschließend wurde bis zur Abkühlung auf Zimmertemperatur gerührt, wobei eine klare leicht viskose Mikroemulsion von sehr schwach gelblicher Färbung entstand.

| Beispiel 9 | |
|---|---|
| Dihydroergotaminmesilat | 3,20 Gew.% |
| Natriumdodecylsulfat | 1,20 Gew.% |
| Tween® 20 | 10,08 Gew.% |
| Cetiol® HE | 15,12 Gew.% |
| Isopropylmyristat | 08,82 Gew.% |
| $\alpha$-Tocopherol | 03,78 Gew.% |
| Wasser, dest. | 57,80 Gew.% |
| Gesamt | 100,00 Gew.% |

Beispiel 8 wurde mit der Ausnahme wiederholt, daß anstelle von Tween® 80 Tween® 20 eingesetzt wurde. Es entstand eine klare leicht viskose Mikroemulsion von sehr schwach gelblicher Färbung.

| Beispiel 10 | |
|---|---|
| Dihydroergotaminmesilat | 3,20 Gew.% |
| Natriumdodecylsulfat | 1,20 Gew.% |
| Tween® 20 | 12,75 Gew.% |
| Cetiol® HE | 19,12 Gew.% |
| Isopropylmyristat | 11,15 Gew.% |
| α-Tocopherol | 04,78 Gew.% |
| Wasser, dest. | 47,80 Gew.% |
| Gesamt | 100,00 Gew.% |

Beispiel 9 wurde mit den aus der obigen Aufstellung ersichtlichen Mengenverhältnissen wiederholt. Es entstand eine klare leicht viskose Mikroemulsion von sehr schwach gelblicher Färbung.

| Beispiel 11 | |
|---|---|
| Atlas® G - 4940 | 18,0 Gew.% |
| Cetiol® HE | 20,0 Gew.% |
| Isopropylmyristat | 5,0 Gew.% |
| Natriumdiclofenac | 2,0 Gew.% |
| Cholinchlorid 75% | 5,8 Gew.% |
| Wasser, dest. | 49,2 Gew.% |
| Gesamt | 100,0 Gew.% |

Die Mischung aus Atlas® G - 4940, Cetiol® HE und Isopropylmyristat wurde unter Rühren auf 90°C erwärmt. Gleichzeitig wurde die Mischung aus Natriumdiclofenac, der 75%igen wäßrigen Cholinchloridlösung und Wasser unter Rühren auf 90°C erwärmt und anschließend unter kräftigem Rühren in die 90°C warme Mischung aus Atlas® G - 4940, Cetiol® HE und Isopropylmyristat eingetragen. Bis zum Abkühlen auf Zimmertemperatur wurde gerührt, wobei ein klares, farbloses, festes Gel entstand.

| Beispiel 12 | |
|---|---|
| Dihydroergotaminmesilat | 3,20 Gew.% |
| Dodecylsulfat | 1,20 Gew.% |
| Tween® 60 | 15,93 Gew.% |
| Cetiol® HE | 15,93 Gew.% |
| Isopropylmyristat | 13,55 Gew.% |
| α-Tocopherol | 2,39 Gew.% |
| Wasser, dest. | 47,80 Gew.% |
| Gesamt | 100,00 Gew.% |

Die Gesamtmenge an Dihydroergotaminmesilat, Dodecylsulfat und Wasser wurden 15 Minuten lang bei Zimmertemperatur gerührt. Anschließend wurde die Gesamtmenge an Tween® 60, Cetiol® HE, Isopropylmyristat und α-Tocopherol hinzugefügt. Die Mischung aus sämtlichen Bestandteilen der Formulierung wurde unter Rühren auf 80°C erwärmt. Anschließend wurde bis zur Abkühlung auf Zimmertemperatur gerührt, wobei eine klare leicht viskose Mikroemulsion von sehr schwach gelblicher Färbung entstand.

| Beispiel 13 | |
|---|---|
| Dihydroergotaminmesilat | 3,2 Gew.% |
| Natriumdioctylsulfosuccinat | 2,2 Gew.% |
| Tween® 80 | 17,1 Gew.% |
| Cetiol® HE | 16,3 Gew.% |
| Isopropylmyristat | 12,7 Gew.% |
| α-Tocopherol | 0,5 Gew.% |
| Wasser, dest. | 48,0 Gew.% |
| Gesamt | 100,00 Gew.% |

Dihydroergotaminmesilat und Natriumdioctylsulfosuccinat wurden 30 Minuten lang in der Gesamtmenge des Wassers bei Zimmertemperatur gerührt. Anschließend wurde die Temperatur unter Rühren auf 55 bis 60° C erhöht, wobei die Suspension ausflockte. Es wurde nun unter kräftigem Rühren die Gesamtmenge von Tween® 80, Cetiol® HE, Isopropylmyristat, und α-Tocopherol hinzugefügt und die Temperatur auf 75 bis 80° C angehoben. Nachdem sich alle Bestandteile aufgelöst hatten, wurde unter Rühren auf Zimmertemperatur abgekühlt.

| Beispiel 14 | |
|---|---|
| Dihydroergotaminmesilat | 3,2 Gew.% |
| Taurocholsäure Na-Salz | 2,5 Gew.% |
| Tween® 80 | 26,0 Gew.% |
| Cetiol® HE | 24,7 Gew.% |
| Isopropylmyristat | 19,5 Gew.% |
| α-Tocopherol | 0,5 Gew.% |
| Wasser, dest. | 23,6 Gew.% |
| Gesamt | 100,00 Gew.% |

Die Gesamtmenge Dihydroergotaminmesilat, Taurocholsäure-Na-Salz, Tween® 80, Cetiol® HE, Isopropylmyristat, und α-Tocopherol wurden 15 Minuten lang bei Zimmertemperatur gerührt. Anschließend wurde die Gesamtmenge Wasser hinzugefügt. Die Mischung aus sämtlichen Bestandteilen der Formulierung wurde unter Rühren auf 80° C erwärmt. Anschließend wurde bis zur Abkühlung auf Zimmertemperatur gerührt, wobei eine opaleszierende leicht viskose Mikroemulsion von schwach gelblicher Färbung entstand.

## Ansprüche

1. Hochdisperse pharmazeutische Zusammensetzung, **dadurch gekennzeichnet,** daß sie als Mikroemulsion vorliegt und durch Mischen von
a) saurem oder basischem Wirkstoff oder dessen Salzen,
b1) in Gegenwart eines sauren Wirkstoffes oder von dessen Salzen einem kationaktiven Ionenpaarbildner, dessen korrespondierende ungeladene Base einen pKs-Wert von mehr als 9 aufweist, in Form seiner Salze oder der korrespondierenden ungeladenen Base,
b2) in Gegenwart eines basischen Wirkstoffes oder von dessen Salzen einem anionaktivem Ionenpaarbildner, dessen korrespondierende ungeladene Säure einen pKs-Wert von weniger als 5 aufweist, in Form seiner Salze oder der korrespondierenden ungeladenen Säure,
c) Isopropylmyristat, 2-Octyldodecanol, Paraffinöl, Tocopherolnicotinat und/oder Triacetin als Fettkomponente,
d) Polyoxyethylenfettsäureester und/oder Polyoxyethylenfettalkoholether als Tensid,
e) Polyoxyethylenglycerinfettsäureester als Cotensid und
f) Wasser
erhältlich ist.
2. Zusammensetzung nach Anspruch 1, **dadurch gekennzeichnet,** daß sie durch Verwendung von Polyoxyethylensorbitanfettsäureester als Tensid erhältlich ist.

3. Zusammensetzung nach Anspruch 1 oder 2, **dadurch gekennzeichnet** daß sie durch Verwendung von Isopropylmyristat als Fettkomponente, Polyoxyethylen-20-sorbitanmonolaurat, -oleat oder -stearat, Polyoxyethylen-30-cetylstearylalkoholether und/oder Polyoxyethylen-30-cetylstearylalkoholether als Tensid und Polyoxyethylen-7-glycerylcocoat als Cotensid erhältlich ist.

4. Zusammensetzung nach einem der Ansprüche 1 bis 3, **dadurch gekennzeichnet, daß** sie durch Verwendung eines Wirkstoffsalzes, das als Gegenion zum Wirkstoffion ein vom Ionenpaarbildner abgeleitetes lipophiles Ion enthält, anstelle von Wirkstoff oder dessen Salzen und Ionenpaarbildner erhältlich ist.

5. Zusammensetzung nach einem der Ansprüche 1 bis 4, **dadurch gekennzeichnet, daß** sie durch Verwendung von Diclofenac und/oder Ibuprofen als saurem Wirkstoff und Mutterkornalkaloiden, $\beta$-Sympathomimetica und/oder $\beta$-Blockern als basischem Wirkstoff erhältlich ist.

6. Zusammensetzung nach Anspruch 5, **dadurch gekennzeichnet, daß** sie durch Verwendung von Dihydroergotaminen, Dihydroergocristin und/oder Dihydroergotoxin als Mutterkornalkaloide, von Salbutamol, Fenoterol, Ipratropium und/oder Terbutalin als $\beta$-Sympathomimetica und von Propranolol, Atenolol, Metoprolol und/oder Acebutolol als $\beta$-Blocker erhältlich ist.

7. Zusammensetzung nach einem der Ansprüche 1 bis 6, **dadurch gekennzeichnet, daß** sie durch Verwendung von mit hydrophilen basischen Gruppen substituierten lipophilen Verbindungen, die ohne basische Gruppe ein Molekulargewicht von mindestens 45 haben, als kationaktive Ionenpaarbildner erhältlich ist.

8. Zusammensetzung nach einem der Ansprüche 1 bis 7, **dadurch gekennzeichnet, daß** sie durch Verwendung von kationaktiven Tensiden und/oder mit hydrophilen basischen Gruppen substituierten lipophilen Verbindungen, die ohne basische Gruppe ein Molekulargewicht von mindestens 50 haben, als kationaktivem Ionenpaarbildner und anionaktiven Tensiden und/oder mit hydrophilen sauren Gruppen substituierten lipophilen Verbindungen, die ohne saure Gruppe ein Molekulargewicht von mindestens 50 haben, als anionaktivem Ionenpaarbildner erhältlich ist.

9. Zusammensetzung nach einem der Ansprüche 1 bis 8, **dadurch gekennzeichnet, daß** sie durch Verwendung von Alkylsulfaten, Alkylpolyglykolethersulfaten, Dialkylsulfosuccinaten, Alkylsulfonaten, Sulfobenzoaten, Salicylsulfaten, Taurocholaten und/oder deren korrespondierenden ungeladenen Säuren als anionaktivem Ionenpaarbildner und Tetraalkylammoniumsalzen, Alkyl-bis-(oligooxyethyl)methylammoniumsalzen, N-Fettsäurealkylamido-N-trimethylammoniumsalzen, Tris-(oligooxyethyl)alkylammoniumsalzen, Estern des Betains, Estern des Cholins mit Fettsäuren und/oder deren korrespondierenden neutralen Basen als kationaktivem Ionenpaarbildner erhältlich ist.

10. Zusammensetzung nach Anspruch 7, **dadurch gekennzeichnet, daß** sie durch Verwendung von Cholinchlorid als kationaktivem Ionenpaarbildner erhältlich ist.

11. Zusammensetzung nach einem der Ansprüche 10, **dadurch gekennzeichnet, daß** sie durch Verwendung eines Gewichtsverhältnisses von Ionenpaarbildner zu Wirkstoff von 0,15 bis 4,0 erhältlich ist.

12. Zusammensetzung nach einem der Ansprüche 1 bis 11, **dadurch gekennzeichnet, daß** sie durch Verwendung eines Gewichtsverhältnisses von Ionenpaarbildner zu Wirkstoff von ungefähr 1 erhältlich ist.

13. Zusammensetzung nach einem der Ansprüche 1 bis 12, **dadurch gekennzeichnet, daß** sie durch Mischen von

a) 0,1 bis 11 Gew.% Wirkstoff oder dessen Salzen,
b) 0,05 bis 12 Gew.% Ionenpaarbildner,
c) 1 bis 25 Gew.% der Fettkomponente,
d) 5 bis 30 Gew.% des Tensids,
e) 5 bis 30 Gew.% des Cotensids und
f) 5 bis 70 Gew.% Wasser
erhältlich ist.

14. Zusammensetzung nach einem der Ansprüche 1 bis 13, **dadurch gekennzeichnet, daß** sie durch Verwendung von Vitamin E-Derivaten als chemischem Stabilisator erhältlich ist.

15. Zusammensetzung nach Anspruch 14, **dadurch gekennzeichnet, daß** sie durch Verwendung von 2 bis 5 Gew.% Vitamin E-Derivaten als chemischem Stabilisator erhältlich ist.

16. Zusammensetzung nach einem der Ansprüche 1 bis 14, **dadurch gekennzeichnet, daß** sie durch Verwendung von $\alpha$-Tocopherol als Vitamin E-Derivat erhältlich ist.

17. Verfahren zur Herstellung der Zusammensetzung nach einem der Ansprüche 1 bis 16, **dadurch gekennzeichnet, daß** der pH-Wert der während des Mischens entstehenden wäßrigen Phasen beim Vorliegen von saurem Wirkstoff oder dessen Salzen im Bereich von pKs-Wert des sauren Wirkstoffs plus 1 bis pKs-Wert der korrespondierenden ungeladenen Base des kationenaktiven Ionenpaarbildners minus 1 und beim Vorliegen von basischem Wirkstoff oder dessen Salzen im Bereich von pKs-Wert der korrespondierenden ungeladenen Säure des anionaktiven Ionenpaarbildners plus 1 bis pKs-Wert des basischen Wirkstoffs minus 1 liegt.

18. Verfahren nach Anspruch 12, **dadurch gekennzeichnet,** daß der pH-Wert der während des Mischens entstehenden wäßrigen Phasen beim Vorliegen von saurem Wirkstoff oder dessen Salzen im Bereich von pKs-Wert des sauren Wirkstoffs plus 2 bis pKs-Wert der korrespondierenden ungeladenen Base des kationaktiven Ionenpaarbildners minus 2 und beim Vorliegen von basischem Wirkstoff oder dessen Salzen im Bereich von pKs-Wert der korrespondierenden ungeladenen Säure des anionaktiven Ionenpaarbildners plus 2 bis pKs-Wert des basischen Wirkstoffs minus 2 liegt.